(19) Europäisches Patentamt
European Patent Office
Office européen des brevets

(11) Veröffentlichungsnummer: **0 245 267**
**B1**

(12) **EUROPÄISCHE PATENTSCHRIFT**

(45) Veröffentlichungstag der Patentschrift: **28.11.90**

(51) Int. Cl.[5]: **C 07 K 7/10**, C 07 K 7/40

(21) Anmeldenummer: **86900761.7**

(22) Anmeldetag: **11.01.86**

(86) Internationale Anmeldenummer:
**PCT/EP86/00008**

(87) Internationale Veröffentlichungsnummer:
**WO 86/04335 31.07.86 Gazette 86/17**

# (54) VERFAHREN ZUR GEWINNUNG VON INSULIN-VORLÄUFERN AUS REAKTIONSGEMISCHEN, DIE BEI DER FALTUNG VON INSULIN-VORLÄUFERN AUS DEN ENTSPRECHENDEN S-SULFONATEN ANFALLEN.

(30) Priorität: **19.01.85 DE 3501641**

(43) Veröffentlichungstag der Anmeldung:
**19.11.87 Patentblatt 87/47**

(45) Bekanntmachung des Hinweises auf die Patenterteilung:
**28.11.90 Patentblatt 90/48**

(84) Benannte Vertragsstaaten:
**AT BE CH DE FR GB IT LI LU NL SE**

(56) Entgegenhaltungen:
**EP-A-0 021 169**
**EP-A-0 037 255**
**EP-A-0 037 256**

**Chemical Abstracts, Band 96, Nr. 21, Mai 1982, Columbus, Ohio (US) R.E. Chance et al.: "The production of human insulin using recombinant DNA technology and a new chain combination procedure", siehe S.749, Zusammfassung 181617g.**

(73) Patentinhaber: **HOECHST AKTIENGESELLSCHAFT**
**Postfach 80 03 20**
**D-6230 Frankfurt am Main 80 (DE)**

(72) Erfinder: **GRAU, Ulrich**
**Am Forsthaus 1**
**D-6238 Hofheim am Taunus (DE)**

(56) Entgegenhaltungen:
**Chemical Abstracts, Band 96, Nr. 21, Mai 1982, Columbus, Ohio (US) B. H. Frank et al.: "The production of human proinsulin and its transformation to human insulin and C-peptide", Seite 749, Zusammenfassung181618h**

**Chemical Abstracts, Band 97, Nr. 13, September 1982, Columbus, Ohio (US) W. Danho et al.: "Syntheses and biological properties of hybrids between human insulin and insulin-like growth factor I", Seite 650, Z'fassung 110370f.**

**Chemical Abstracts, Band 95, Nr. 25, Dez. 1981, Columbus, Ohio, (US) G.Losse et al.: "New hybrid insulins with synthetically sequence-modified B chains", Seite 539, Z'fassung 220286z**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 245 267 B1

(56) Entgegenhaltungen:

**Tetrahedron Letters, Nr. 12, 1973, Pergamon Press (GB) S M L Robinson et al.:"Spaltung und Rückbildung der Disulfidbrücken an intramolekular vernetzten Insulinen", S. 985-988, siehe da ganze Dokument.**

**Chemical Abstracts, Band 95, 1981, Columbus, Ohio, US Naithani, Vinod K. et al.: "Semisynthesis of human proinsulin.I. Preparation of arginyl-A-chain cyclic bisdisulfide", Seite 540, Z'fassung 220291x .**

**Beschreibung**

Es ist ein Verfahren zur Herstellung eines Insulin-Vorläufers der Formel I (siehe Patentanspruch) bekannt, worin R Wasserstoff, ein chemisch oder enzymatisch abspaltbarer Aminosäurerest oder ein chemisch oder enzymatisch abspaltbarer Peptidrest mit wenigstens zwei Aminosäureresten ist, Y für $—Lys^{B29}—Z^{B30}—$ steht, worin Z Ala, Thr oder Ser bedeutet, wobei die sich von A-1 bis A-21 erstreckende Brücke eine Insulin-A-Kette ist, die sich von B-1 bis B-30 erstreckende Brücke eine Insulin-B-Kette darstellt und X eine Brücke ist, welche an der Aminogruppe von A-1 an die Insulin-A-Kette und an der Carboxylgruppe von B-30 an die Insulin-B-Kette gebunden ist, wobei diese Brücke enzymatisch oder chemisch ohne Zerstörung der A-Kette und der B-Kette gespalten werden kann.

Bei diesem Verfahren setzt man ein S-Sulfonat der Formel II (siehe Patentanspruch 1), worin R, X und Y die oben angegebenen Bedeutungen haben, in einem wäßrigen Medium bei einem pH-Wert von 7 bis 11,5 und bei einer S-Sulfonatkonzentration von bis zu 10 mg pro ml des wäßrigen Mediums mit einer solchen Menge eines Mercaptans um, die 1—5 SH-Gruppen je $SSO_3^-$ -Einheit ergibt (vgl. Europäische Patentschrift 37 255 entsprechend jap. OS 81—150051 und US—PS 4 430 266). Die Faltungsausbeute ist bei diesem Verfahren von mehreren Parametern, wie pH, Verhältnis $SSO_3^-$ zu —SH, der Konzentration, der Art des Mercaptans, der Temperatur und der Reaktionsdauer abhängig. Unter praxisnahen Bedingungen kann man das Verfahren auf eine Faltungsausbeute von etwa 60% optimieren.

Mit anderen Worten handelt es sich hierbei um die Herstellung eines Insulinvorläufers durch Faltung unter Ausbildung der nativen Raumstruktur mit drei Disulfidbrücken, aus dem dann durch anschließende Umwandlung mit Proteasen das freie Insulin gewonnen werden kann. Bei der Umsetzung mit den Proteasen werden die als Nebenprodukte entstehenden falsch verknüpften Insulinvorläufer in Bruchstücke zerlegt, die mit Hilfe des gleichen Verfahrens nicht mehr zu den erwünschten Insulinvorläufern aufgearbeitet werden können. Unter dem Begriff Insulinvorläufer werden hierbei sowohl Proinsuline als auch Prä-Proinsuline verstanden, wobei sich die Vorsilbe "Prä" auf eine oder mehrere zusätzliche Aminosäuren am N-Terminus des Proinsulins beziehen soll und wobei der Proinsulin-Teil selbst vorzugsweise die Sequenz des Human- oder Affen-Proinsulins aufweist. Prinzipiell sind natürlich auch andere Proinsuline möglich, z.B. des Schweins, Rinds, Schafs, die aus Pankreas isolierbar sind, oder solche mit synthetischen Sequenzen, die die Humaninsulinsequenz enthalten und die nach gentechnologischen Verfahren hergestellt bzw. im Fall von Schweineinsulin semisynthetisch zum Humaninsulin aufgearbeitet werden können.

Bekanntlich sind nicht alle Disulfidbrücken des Insulins gleich reaktiv, sondern bei milder Reduktion des Disulfids wird zunächst die Disulfidbrücke zwischen den Cysteinen A7 und B7 geöffnet, bevor die zweite Disulfidbrücke zwischen den Cysteinen B19 und A20 geöffnet wird. Die bevorzugte Reduktion der A7—B7-Disulfidbrücke wurde in eigenen Untersuchungen auch bei der Reaktion von Insulinvorläufer-S-Sulfonat mit geringem Überschuß an Mercaptan beobachtet.

Die weitere Aufarbeitung des gefalteten Insulin-Vorläufers erfolgt nach der Literatur mit chromatographischen Verfahren, z.B. durch Entsalzung mittels Gelchromatographie, z.B. an (R) Sephadex G 25, gefolgt von Gelchromatographie an Sephadex G50 superfine, wobei in der zweiten Stufe "aggregierte Formen" von nativen Insulinvorläufern getrennt werden.

Es wurde nun überraschend gefunden, daß die bei der Faltung von Insulin-Vorläufern aus den entsprechenden S-Sulfonaten anfallenden falschen Rekombinanten, deren Anteil gewöhnlich 30 bis 50% der eingesetzten Menge ausmacht, direkt aus dem Faltungsmedium bei pH 4 bis 6 in Gegenwart einer kleinen Menge einer physiologisch unbedenklichen Oberflächenaktiven Substanz ausgefällt werden können, wohingegen die native Form des Insulinvorläufers nahezu vollständig in Lösung bleibt. Dies gilt sowohl, wenn zur Faltung etwa 2 Äquivalente —SH pro S-Sulfonatgruppe eingesetzt werden als auch, wenn eine etwas größere Menge an Mercaptan eingesetzt wird, was unter weitergehender Reduktion zu Cysteingruppen und damit zur Bildung solcher Insulinvorläufer führt, die nur partiell Disulfidbrücken enthalten. Der Niederschlag wird dann in üblicher Weise, z.B. durch Zentrifugieren, abgetrennt und durch Sulfitolyse in das S-Sulfonat der Formel II übergeführt. Dieses wird dann erneut der Faltung unterworfen.

Bei den erfindungsgemäß hergestellten Verbindungen der Formel I ist R Wasserstoff oder ein chemisch oder enzymatisch abspaltbarer Aminosäurerest oder ein chemisch oder enzymatisch abspaltbarer Peptidrest mit wenigstens 2 Aminosäureresten der Formel $P_m—Q_n—$; darin ist P eine Sequenz natürlich vorkommender Aminosäuren mit m von 0 bis 50 und Q stellt basische natürliche Aminosäuren, insbesondere Arginin oder Lysin, mit n=1 bis 4 dar. $P_m$ kann insbesondere eine Teilsequenz der β-Galaktosidase sein, wobei das entsprechende Fragment $P_m$ direkt exprimiert oder erst durch Bromcyanspaltung zustande gekommen sein kann. Y steht für $—Lys^{B29}—Z^{B30}—$, worin Z Ala, Thr oder Ser bedeutet; die sich von A-1 bis A-21 erstreckende Brücke ist eine Insulin-A-Kette, die sich von B-1 bis B-30 erstreckende Brücke eine Insulin-B-Kette und X eine Brücke, welche an der Aminogruppe von A-1 an die Insulin-A-Kette und an der ε-Aminogruppe von B-29 — in welchem Falle an die freie Bindung von $Z^{B30}$ OH gebunden ist — oder an der Carboxylgruppe von B-30 an die Insulin-B-Kette gebunden ist.

Das erfindungsgemäße Verfahren ermöglicht es nun, die Gesamt-Faltungsausbeute dadurch zu steigern, daß der Anteil an falschen Rekombinanten nunmehr nicht mehr verlorengeht, sondern in einem einfachen Verfahren zu dem erwünschten Insulinvorläufer aufgearbeitet wird. Durch die Gegenwart einer kleinen Menge einer physiologisch unbedenklichen Oberflächenaktiven Substanz wird insbesondere die

Adsorption von nativ gefalteten Insulinvorläufern an die falschen Rekombinanten verhindert. Geeignete physiologisch unbedenklichen, oberflächenaktiven Substanzen sind z.B. Polymerisate, d.h. Homopolymerisate Mischpolymerisate oder Blockpolymerisate, der Formel $R^2$—O—$X_n$—$R^3$ (III), in der $X_n$ eine Kette von n Gliedern der Formel —CH($R^1$)—CH($R^1$)—O— (IV) in beliebiger Reihenfolge, n 2 bis 80, vorzugsweise 15 bis 45, und $R^1$ Wasserstoff, —$CH_3$ oder —$C_2H_5$ ist, wobei die Reste $R^1$ gleich oder verschieden sein können und $R^2$ und $R^3$ unabhängig voneinander Wasserstoff oder ein organischer Rest sind, jedoch mit der Maßgabe, daß die Verbindungen III mindestens 12 C-Atome enthalten. $R^2$ und $R^3$ können z.B. Alkyl mit 1 bis 20 C-Atomen, Carboxyalkyl mit 2 bis 20 C-Atomen oder Alkylphenyl mit 1 bis 10 Alkyl-C-Atomen bedeuten. Beispiele für die Reste $R^2$ und $R^3$ sind Methoxy, Äthoxy, Propoxy, Butoxy oder die Reste, die sich von Lauryl-, Myristyl- oder Cetylalkohol ableiten; Carboxyalkylgruppen, die sich von der Essig, Propion-, Butter-, Palmitin- oder Stearinsäure ableiten, Nonylphenoxy, Oleylamino oder Stearylamino.

$R^2$ oder $R^3$ kann sich auch von einem mehrwertigen Alkohol wie Glycerin oder Pentaerytrit oder einer mehrwertigen Carbonsäure wie Citronensäure ableiten. Mehrfunktionelle Glieder $R^2$ oder $R^3$ können mit zwei oder mehreren Polyalkoxyketten der oben gezeigten Art verbunden sein, wobei verzweigte Produkte entstehen.

Die vorgenannten oberflächenaktiven Substanzen, die im allgemeinen in einer Menge von 1 bis 400, vorzugsweise 2 bis 200 mg/l zugesetzt werden, lassen sich in üblicher Weise durch kontrollierte Addition von Alkylenoxiden an Alkylenpolyglykole herstellen. Die endständigen Hydroxylfunktionen können gegebenenfalls anschließend verestert oder veräthert werden.

Als oberflächenaktive Substanzen können ferner Phosphorlipide der Formel

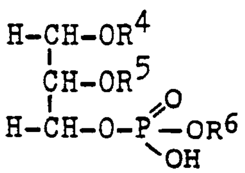

(V)

verwendet werden, in der $R^4$ und $R^5$, die gleich oder verschieden sein können, für Alkylcarbonyl, Alkenylcarbonyl, Alkandienylcarbonyl, Alkantrienylcarbonyl mit jeweils 8 bis 22, vorzugsweise 12 bis 22 C-Atomen oder Wasserstoff stehen, mit der Maßgabe, daß $R^4$ und $R^5$ nicht gleichzeitig Wasserstoff sind, und in der $R^6$ für eine hydrophile Gruppe steht. Die Konzentration dieser Verbindungen beträgt im allgemeinen 1 bis 20, vorzugsweise 1 bis 10, insbesondere 2,5 bis 7,5 Gewichts-%. Beispiele solcher hydrophilen Gruppen sind 2-(Trimethylammonium)äthyl, 2-Aminoäthyl, 2-Carboxy-2-aminoäthyl, 2,3-Dihydroxypropyl oder 2,3,4,5,6-Pentahydroxycyclohexyl. Bevorzugt sind Verbindungen, in denen $R^4$ und $R^5$ je für Alkylcarbonyl stehen. Weiterhin bevorzugt sind Verbindungen, in denen $R^6$ für 2-(Trimethylammonium)- äthyl steht, wobei solche Verbindungen als Lecithine bekannt sind und solche Verbindungen, in denen $R^4$ und $R^5$ je für Alkylcarbonyl mit etwa 8 bis 16 C-Atomen oder mit etwa 12 bis 16 C-Atomen und in denen $R^6$ für 2-(Trimethylammonium)äthyl stehen, insbesondere solche Verbindungen, in denen $R^4$ und $R^5$ je für Octanoyl stehen.

Weiterhin kommen als oberflächenaktive Substanzen Verbindungen der Formel $R^7$—[—O—$CH_2$—$CH_2$—O$]_p$—H (VI) in Frage, worin $R^7$ eine gesättigte oder olefinisch ungesättigte Kohlenwasserstoffgruppe mit 8 bis 15 C-Atomen und p eine ganze Zahl von 2 bis 25 bedeutet. Diese Verbindungen werden im allgemeinen in einer Menge von 0,2 bis 200 mg/l zugesetzt. $R^7$ ist vorzugsweise ($C_{12}$ oder $C_{13}$)-Alkyl, p vorzugsweise 4 bis 23, insbesondere 6 bis 15.

Bei dem erfindungsgemäßen Verfahren wendet man im allgemeinen die Faltungsbedingungen an, die in der europäischen Patentschrift 37 255 beschrieben sind. Das heißt, daß man im allgemeinen bei einer Temperatur von 0 bis 37°C, mit 1 bis 5 —SH-Einheiten je —$SO_3$- Gruppe in einem pH-Bereich von 7 bis 11,5 und einer S-Sulfonat-Insulinvorläufer-Konzentration von bis zu 10 mg je ml von wäßrigem Medium arbeitet. Bevorzugte Mercaptane sind 2-Mercaptoäthanol, Thioglykolsäure bzw. deren Methylester, 3-Mercapto-1,2-propandiol und 3-Mercaptopropionsäure bzw. deren Ester. Bevorzugte Reaktionsbedingungen sind eine Konzentration von 0,2 bis 1 mg/ml S-Sulfonat-Insulinvorläufer, ein pH-Bereich von 9,5 bis 11, eine Reaktionstemperatur von 2 bis 10°C, eine Reaktionsdauer von 5 bis 20 Stunden und ein Verhältnis von —SH zu —$SO_3$- -Gruppe von 2—3. Bevorzugt ist aus praktischen Gründen auch der weitgehende Ausschluß von Sauerstoff, wenngleich er keineswegs notwendig ist.

Die pH-Umstellung wird vorteilhaft durch Zugabe von verdünnten Mineralsäuren, wie Salz-, Schwefel- oder Phosphorsäure bewirkt, jedoch kann man auch organische Säuren, die mit den Insulinvorläufern nicht reagieren, wie Essigsäure, oder saure Salze, wie Phosphate verwenden.

Der bei der pH-Umstellung erhaltene Niederschlag kann leicht abzentrifugiert werden. Er wird durch direkte Sulfitolyse, wie sie z.B. von Swan in Nature *180*, 643—645 (1957) beschrieben ist, weitgehend wieder in die S-Sulfonatform übergeführt. Diese kann dann gegebenenfalls durch chromatographische Verfahren gereinigt und dann z.B. in der oben angegebenen Weise, vorteilhaft in Gegenwart einer kleinen Menge einer physiologisch unbedenklichen oberflächenaktiven Substanz der genannten Art, in den

gefalteten Insulinvorläufer übergeführt werden. Sie kann aber auch nach einer weiteren Ausführungsform aufgearbeitet werden, nämlich indem man partiell verknüpften Insulinvorläufern, die mit einem geringen Überschuß an Mercaptan hergestellt worden sind, eine oberflächenaktive Substanz der oben genannten Art zusetzt, Sauerstoff, z.B. Luftsauerstoff, durch die Lösung perlt und so die Reoxydation bewerkstelligt. Die oberflächenaktive Substanz verhindert dabei die Denaturierung des Insulinvorläufers, z.B. an den Gasblasen.

Durch das erfindungsgemäße Verfahren läßt sich also die Faltungsausbeute, einschließlich der Wiedergewinnung falscher Rekombinanten, auf 80 oder bis sogar 90% steigern.

## Beispiele

1. Herstellung von humanen Präproinsulin aus Präproinsulin-S-Sulfonat

1,2 g Präproinsulin-S-Sulfonat mit der Präsequenz R = Gly-Asn-Ser-Ala-Arg- und der Sequenz von humanem Proinsulin wurden in 2,5 l 50 mM entgastem Glycinpuffer vom pH 10,6 gelöst und mit 147 µl Mercaptoäthanol über Nacht bei 4°C schwach gerührt. Nach der Hochdruckflüssigkeitschromatographie (High Performance Liquid Chromatography = HPLC)-Analyse betrug die Faltungsausbeute im Medium 0,32 mg/ml (67%) und mit Staphylococcus aureus (Protease V8) Fingerprint wurde ermittelt, daß die A7—B7 Disulfidbrücke zu 65% und die B19—A20 Disulfidbrücke zu 93% vorliegt. Die Fingerprint-Analyse wurde wie folgt durchgeführt:

Zu 200 µl einer Lösung von 0,5 mg/ml des Insulinvorläufers in 50 mM Tris-(hydroxymethyl)-methyl-ammoniumchlorid(Tris) pH 7,5 wurden 0,1 Units S. aureus Protease V8 gegeben und die Lösung 30 Minuten bei 37°C inkubiert. Ein Aliquot der Lösung wird dann direkt in der HPLC analysiert. Die HPLC-Analyse wurde an C18 Umkehrphase mit einem linearen Gradienten von 20 bis 45% B durchgeführt. Als Puffer A diente 0,05 M Tetraäthylammoniumphosphat, 0,25 M Natriumperchlorat und 10% Acetonitril in der Gesamtphase (pH 3). Als Puffer B diente 0,05 M Tetraammoniumphosphat mit 90% Acetonitril in der Gesamtphase (pH 3). Aus dem Verhältnis der auftretenden Fragment-Peaks bei etwa 7, 10 und 14 Minuten und unter Berücksichtigung eines Standards von Human-Proinsulin wurden die obengenannten Werte von 65 und 93% ermittelt.

Die Lösung wurde auf pH = 9 gebracht und 25 mg grenzflächenaktives Polyäthylen-polypropylenglykol zugesetzt. Dann ließ man bei 4°C unter Rühren innerhalb einer Stunde etwa 50 l Sauerstoff durchperlen.

Die Lösung wurde mit 1 N HCl auf pH = 4,5 gebracht, wobei eine Trübung auftritt, die abzentrifugiert wurde. Der Niederschlag wurde mit Aceton und Diäthyläther oder Methyl-tert.butyläther gewaschen und getrocknet; Auswaage: 520 mg. Aus dem klaren Überstand wurde das native Präproinsulin durch Zugabe von 250 g NaCl bei pH = 3 ausgefällt. Ausbeute: 720 mg (60%).

Der bei pH 5,4 ausgefallene Niederschlag wurde in 100 ml 7M Harnstofflösung mit 800 mg Natriumsulfit und 600 mg Natriumtetrathionat bei pH 7,6 bei 4°C sulfitolysiert und das Reaktionsprodukt durch Dialyse gegen wäßrigen Puffer (pH 3) ausgefällt. Die Auswaage des getrockneten Niederschlags ergab 540 mg, wobei der Anteil an zurückgewonnenem Präproinsulin-S-sulfonat 382 mg betrug. Dieses wurde mit 40 µl Mercaptoäthanol wie zuvor angegeben, reduziert, wobei nochmals 226 mg natives Präproinsulin entstanden (individuelle Ausbeute auf dieser Stufe 59%), so daß die kumulierte Faltungsausbeute 946 mg, entsprechend 78,8% betrug.

In einer zweiten, analogen Wiederholung wurden aus den bei der ersten Wiederholung angefallenen 144 mg durch Fällung bei pH = 5,4 nochmals 78 mg (individuelle Ausbeute in der dritten Stufe 54%) natives Präproinsulin gewonnen. Die kumulierte Ausbeute an nativem Präproinsulin betrug jetzt 1,024 g, entsprechend 85,3%.

2. Rückfaltung von Proinsulin vom Schwein

0,4 g Schweine-Proinsulin-S-sulfonat wurden in 800 ml 50 mM Glycinpuffer von ca. 4°C von pH 10,5 gelöst, die Lösung entgast und 49,1 mg Thioglykolsäure zugegeben. Die Lösung wurde über Nacht bei 4°C schwach gerührt. Die Lösung wurde dann mit 4N HCl auf pH 5,2 gestellt, wobei ein Niederschlag ausfiel. Dieser wurde abzentrifugiert, mit Aceton und Dialkyläther gewaschen und getrocknet. Auswaage: 115 mg. Der klare Überstand enthielt nach der HPLC 239 mg (Ausbeute 60%) Proinsulin.

Der Niederschlag wurde wie im Beispiel 1 beschrieben sulfitolysiert, wobei 120 mg anfielen, aus denen gemäß der vorgenannten Behandlung 88 mg Proinsulin-S-sulfonat gewonnen wurden. Diese wurden mit 12,3 mg Thioglykolsäure analog wie in der ersten Stufe rückgefaltet. Bei pH 5,2 bildete sich eine schwache Trübung, die nach Zentrifugieren nochmals 43 mg trockenes Material ergab.

Der beim Zentrifugieren erhaltene Überstand, der 55 mg Proinsulin enthielt (62% Ausbeute, entsprechend 73,5% kumulative Ausbeute), wurde mit dem bei pH 5,2 erhaltenen Überstand aus der ersten Stufe vereint (1,0 l). Es wurden 6 g Tris zugegeben, auf pH 7,2 eingestellt und 0,6 U Trypsin und 6 U Carboxypeptidase B zugegeben. Die Lösung wurde bei 25°C schwach gerührt und der Fortgang der Reaktion in der HPLC verfolgt. Nach etwa 3—4 Stunden war das Proinsulin vollständig verschwunden und statt-dessen C-Peptid und Schweineinsulin entstanden.

Die Reaktion wurde durch Zugabe von 40 ml 1%iger $ZnCl_2$-Lösung und Einstellen auf pH 5,4 beendet. Es fiel ein flockiger, leicht zentrifugierbarer Niederschlag an, der weitgehend aus Schweineinsulin bestand. Ausbeute 201 mg (91% bezogen auf eingesetztes rückgefaltetes Proinsulin). Die Hochreinigung des so isolierten Schweineinsulins erfolgte nach üblichem Verfahren, z.B. an Ionenaustauscher.

**Patentansprüche**

1. Verfahren zur Gewinnung von Insulinvorläufern der Formel (I)

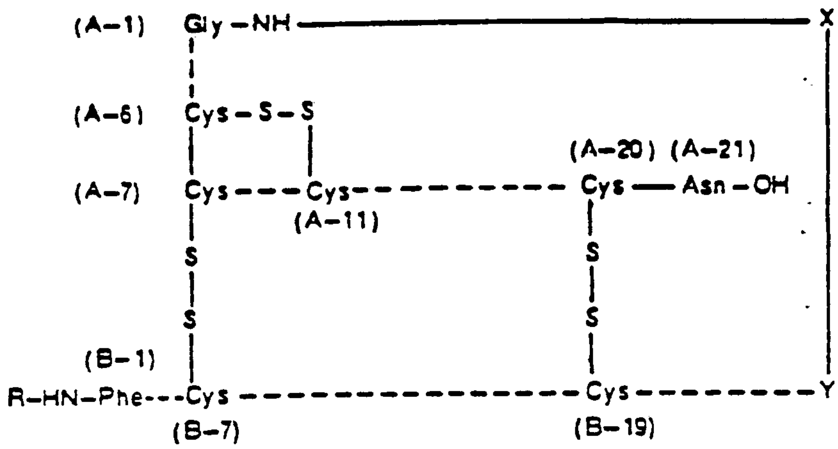

(I)

worin R Wasserstoff oder die Präsequenz $P_m$—$Q_n$— ist, in der P eine Sequenz natürlich vorkommender Aminosäuren mit m von 0 bis 50, Q basische natürliche Aminosäuren und n eine ganze Zahl von 1 bis 4 sind, Y für —$Lys^{B29}$—$Z^{B30}$— steht, worin Z Ala, Thr oder Ser bedeutet, und die sich von A-1 bis A-21 erstreckende Brücke eine Insulin-A-Kette ist, die sich von B-1 bis B-30 erstreckende Brücke eine Insulin-B-Kette darstellt und X eine Brücke ist, welche an der Aminogruppe von A-1 an die Insulin-A-Kette und an der ε-Aminogruppe von B-29 — in welchem Falle an die freie Bindung von $Z^{B30}$ OH gebunden ist — oder an der Carboxylgruppe von B-30 an die Insulin-B-Kette gebunden ist, aus Reaktionsgemischen, die bei der Faltung von Insulinvorläufern aus S-Sulfonaten der Formel (II)

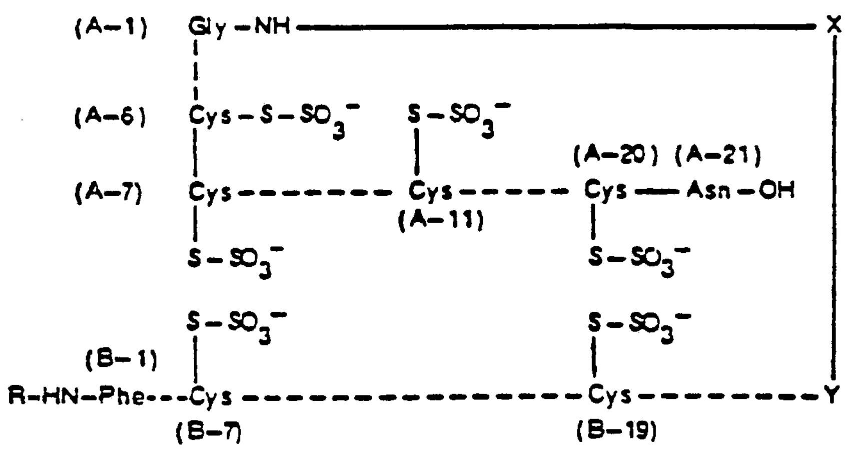

(II)

anfallen, worin R, X und Y die oben angegebenen Bedeutungen haben, dadurch gekennzeichnet, daß die imReaktionsgemisch enthaltenen falschen Rekombinanten durch Einstellung des Reaktionsgemisches auf pH 4 bis 6 in Gegenwart einer kleinen Menge einer physiologisch unbedenklichen oberflächenaktiven Substanz ausgefällt werden, der Niederschlag in üblicher Weise abgetrennt und durch Sulfitolyse in das S-Sulfonat der Formel (II) übergeführt wird und dieses dann erneut der Faltung unterworfen wird.

2. Verfahren nach Anspruch 1, dadurch gekennzeichnet, daß die oberflächenaktive Substanz ein Polymerisat der Formel $R^2$—O—$X_n$—$R^3$ (III) ist, in der $X_n$ eine Kette von n Gliedern der Formel

$$—CH(R^1)—CH(R^1)—O— \qquad (IV),$$

n 2 bis 80, vorzugsweise 15 bis 45, und $R^1$ Wasserstoff, Methyl oder Äthyl ist, wobei $R^2$ und $R^3$ unabhängig voneinander Wasserstoff oder ein organischer Rest sind, jedoch mit der Maßgabe, daß die Verbindungen III mindestens 12 C-Atome enthalten.

3. Verfahren nach Anspruch 2, dadurch gekennzeichnet, daß das Polymerisat in einer Menge von 1 bis 400, vorzugsweise 2 bis 200 mg/l zugesetzt wird.

4. Verfahren nach einem oder mehreren der Ansprüche 1 bis 3, dadurch gekennzeichnet, daß die Faltung in Gegenwart einer kleinen Menge einer physiologisch unbedenklichen oberflächenaktiven Substanz durchgeführt wird.

5. Verfahren nach Anspruch 4, dadurch gekennzeichnet, daß bei der Faltung ein geringer Überschuß an Mercaptan eingesetzt wird und die Reoxydation mit Sauerstoff durchgeführt wird.

6. Verfahren nach einem oder mehreren der Ansprüche 1 bis 5, dadurch gekennzeichnet, daß die Faltung unter Anwendung eines Verhältnisses von —SH-Gruppen zu —$SSO_3^-$-Gruppen von 1 bis 5 in einem wäßrigen Medium bei einem pH von 7 bis 11,5 und einer S-Sulfonat-Insulinvorläuferkonzentration von bis zu 10 mg je ml und bei einer Temperatur von 0 bis 37°C durchgeführt wird.

7. Verfahren nach Anspruch 6, gekennzeichnet durch wenigstens eines der Merkmale, daß die Reaktion mit 0,2 bis 1 mg S-Sulfonat-Insulinvorläufer-Konzentration je ml wäßrigem Medium, bei einem pH von 9,5 bis 11, bei einer Reaktionstemperatur von 2 bis 10°C, bei einer Reaktionsdauer von 5 bis 20 Stunden und bei Anwendung eines Verhältnisses von —SH-Gruppen zu —$SSO_3^-$-Gruppen von 2 bis 3 und bei weitgehendem Ausschluß von Sauerstoff durchgeführt wird.

8. Verfahren nach einem oder mehreren der Ansprüche 1 bis 7, dadurch gekennzeichnet, daß das Mercaptan 2-Mercaptoäthanol, Thioglykolsäure bzw. deren Methylester, 3-Mercapto-1,2-propandiol oder 3-Mercaptopropionsäure bzw. deren Ester ist.

**Revendications**

1. Procédé pour l'obtention de précurseurs d'insuline de formule (I)

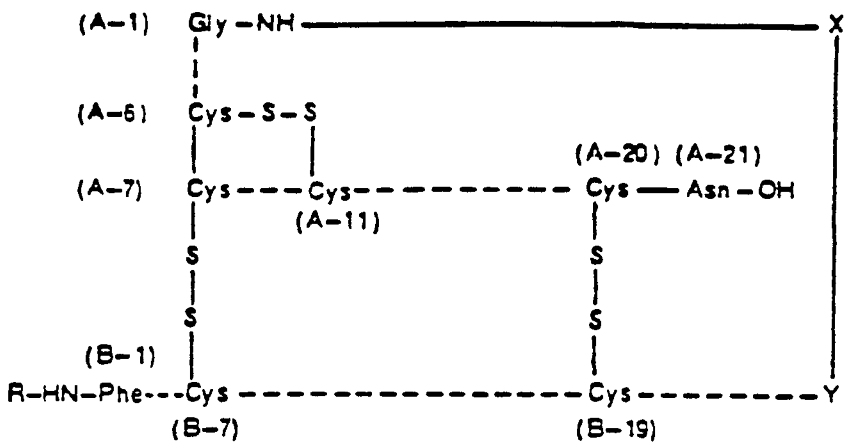

(I)

dans laquelle R est un atome d'hydrogène ou la séquence "pré" $P_m$—$Q_n$—, dans laquelle P est une séquence d'aminoacides existant dans la nature, m allant de 0 à 50, Q représente des aminoacides naturels basiques et n est un nombre entier allant de 1 à 4, Y représente —$Lys^{B29}$—$Z^{B30}$—, Z représentant Ala, Thr ou Ser, et le pont s'étendant de A-1 à A-21 est une chaîne A d'insuline, le pont s'étendant de B-1 à B-30 est une chaîne B d'insuline, et X est un pont qui est lié au groupe amino de A-1 sur la chaîne A de l'insuline et au groupe ε-amino de B-29 — auquel cas OH est fixé sur la liaison libre de $Z^{B30}$ — ou au groupe carboxy de B-30 sur la chaîne B de l'insuline, à partir de mélanges réactionnels qui sont formés lors du repliement de précurseurs d'insuline constitués de S-sulfonates de formule (II)

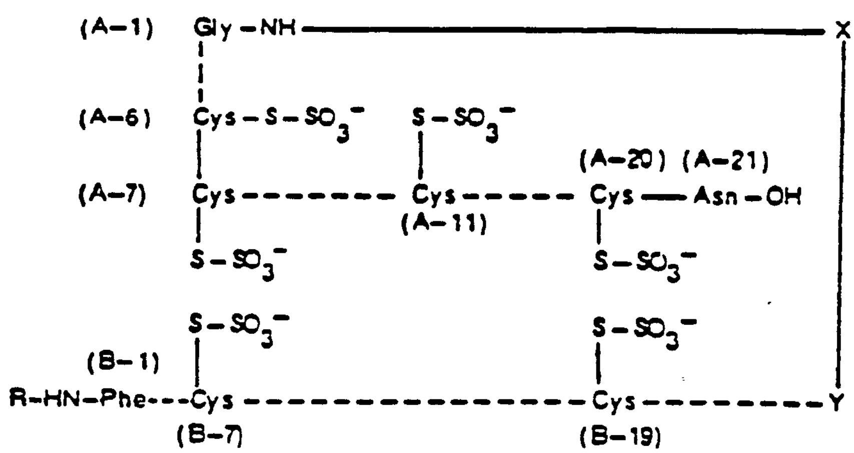

(II)

dans laquelle R, X et Y ont les significations indiquées plus haut, caractérisé en ce que l'on fait précipiter les recombinants incorrects contenus dans le mélange réactionnel, par ajustement du mélange réactionnel à pH 4—6 en présence d'une faible quantité d'une substance tensioactive physiologiquement acceptable, on

sépare le précipité à la manière usuelle et on le convertit par sulfitolyse en le S-sulfonate de formule (II), et ensuite on soumet à nouveau celui-ci au repliement.

2. Procédé selon la revendication 1, caractérisé en ce que la substance tensioactive est un polymère de formule $R^2—O—X_n—R^3$ (III), dans laquelle $X_n$ est une chaîne à n chaînons de formule

$$—CH(R^1)—CH(R^1)—O— \qquad (IV)$$

n allant de 2 à 80, de préférence de 15 à 45, et $R^1$ est un atome d'hydrogène ou le groupe méthyle ou éthyle, $R^2$ et $R^3$ étant, indépendamment l'un de l'autre, un atome d'hydrogène ou un radical organique, avec toutefois la condition que les composés III contiennent au moins 12 atomes de carbone.

3. Procédé selon la revendication 2, caractérisé en ce que le polymère est ajouté en quantité de 1 à 400, de préférence de 2 à 200 mg/l.

4. Procédé selon une ou plusieurs des revendications 1 à 3, caractérisé en ce que le repliement est effectué en présence d'une faible quantité d'une substance tensioactive physiologiquement acceptable.

5. Procédé selon la revendication 4, caractérisé en ce que l'on utilise un faible excès de mercaptan lors du repliement et on effectue la réoxydation avec de l'oxygène.

6. Procédé selon une ou plusieurs des revendications 1 à 5, caractérisé en ce que l'on effectue le repliement en utilisant un rapport des groupes —SH aux groupes $—SSO_3^-$ allant de 1 à 5, dans un milieu aqueux, à un pH de 7 à 11,5 et à une concentration de précurseur d'insuline S-sulfonate allant jusqu'à 10 mg par ml, et à une température de 0 à 37°C.

7. Procédé selon la revendication 6, caractérisé par au moins l'une des caractéristiques que la réaction est effectuée avec une concentration de précurseur d'insuline S-sulfonate allant de 0,2 à 1 mg par ml de milieu aqueux, à un pH de 9,5 à 11, à une température de réaction de 2 à 10°C, pendant une durée de réaction de 5 à 20 heures et en utilisant un rapport des groupes —SH aux groupes $—SSO_3^-$ allant de 2 à 3, et avec exclusion dans une large mesure de l'oxygène.

8. Procédé selon une ou plusieurs des revendications 1 à 7, caractérisé en ce que le mercaptan est le 2-mercaptoéthanol, l'acide thioglycolique ou son ester méthylique, le 3-mercapto-1,2-propanediol ou l'acide 3-mercaptopropionique ou ses esters.

## Claims

1. A process for the isolation of insulin precursors of the formula (I)

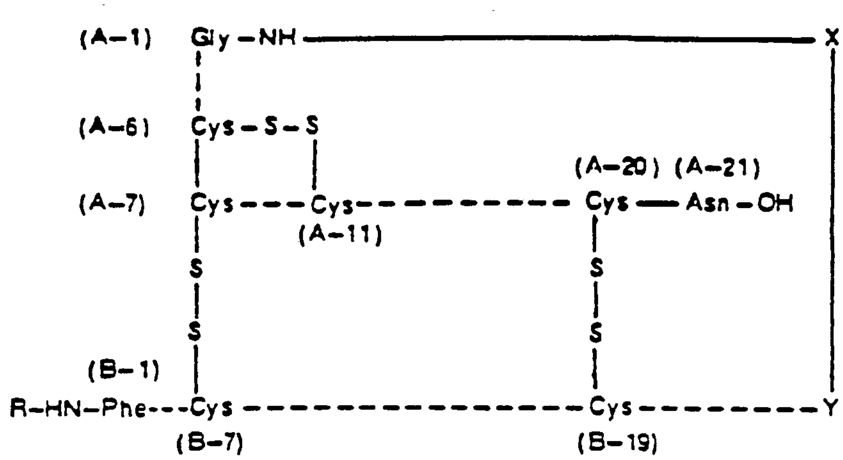

(I)

in which R is hydrogen or the presequence $P_m—Q_n—$, in which P is a sequence of naturally occurring amino acids with m being from 0 to 50, Q are basic natural amino acids, and n is an integer from 1 to 4, Y represents $—Lys^{B29}—Z^{B30}—$, in which Z denotes Ala, Thr or Ser, and the bridge extending from A-1 to A-21 is an insulin A-chain, the bridge extending from B-1 to B-30 represents an insulin B-chain, and X is a bridge which is bonded to the insulin A-chain at the amino group of A-1 and is bonded to the insulin B-chain at the ε-amino group of B-29 — in which case it is bonded to the free bond of $Z^{B30}OH$ — or at the carboxyl group of B-30, from reaction mixtures which result from folding of insulin precursors from S-sulfonates of the formula (II)

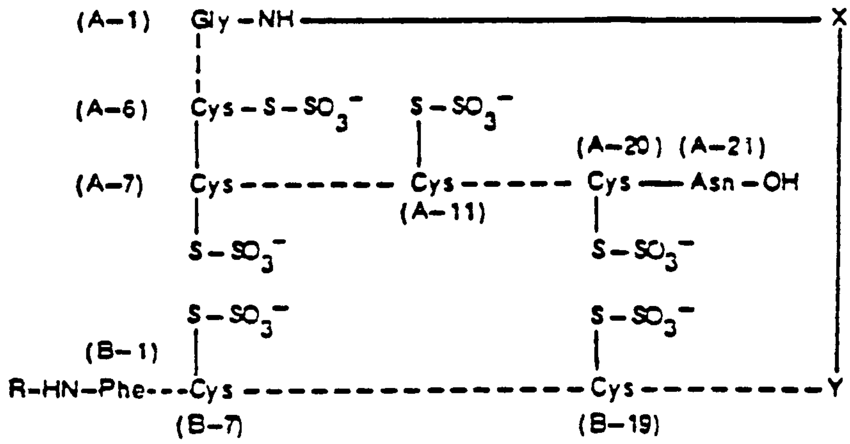

(II)

in which R, X and Y have the abovementioned meanings, which comprises the false recombinants contained in the reaction mixture being precipitated by adjustment of the reaction mixture to pH 4 to 6 in the presence of a small quantity of a physiologically acceptable surface-active substance, the precipitate being removed in customary manner and converted by sulfitolysis into the S-sulfonate of the formula (II), and the latter then being subjected to renewed folding.

2. The process as claimed in claim 1, wherein the surface-active substance is a polymer of the formula $R^2—O—X_n—R^3$ (III), in which $X_n$ is a chain of n members of the formula

$$—CH(R^1)—CH(R^1)—O— \qquad (IV),$$

n is 2 to 80, preferably 15 to 45, and $R^1$ is hydrogen, methyl or ethyl, and $R^2$ and $R^3$ being, independently of one another, hydrogen or an organic radical, but with the proviso that the compounds III contain at least 12 carbon atoms.

3. The process as claimed in claim 2, wherein the polymer is added in an amount of 1 to 400, preferably 2 to 200 mg/l.

4. The process as claimed in one or more of claims 1 to 3, wherein the folding is carried out in the presence of a small quantity of a physiologically acceptable surface-active substance.

5. The process as claimed in claim 4, wherein a small excess of mercaptan is used for the folding, and the reoxidation is carried out with oxygen.

6. The process as claimed in one or more of claims 1 to 5, wherein the folding is carried out using a ratio of —SH groups to $—SO_3^-$ groups of 1 to 5, in an aqueous medium, at a pH of 7 to 11.5 and a S-sulfonate insulin precursor concentration of up to 10 mg per ml, and at a temperature of 0 to 37°C.

7. The process as claimed in claim 6, wherein at least one of the following applies: the reaction is carried out with a S-sulfonate insulin precursor concentration of 0.2 to 1 mg per ml of aqueous medium, at a pH of 9.5 to 11, at a reaction temperature of 2 to 10°C, with a reaction time of 5 to 20 hours, and using a ratio of —SH groups to $—SSO_3^-$ groups of 2 to 3, and with exclusion of oxygen to a large extent.

8. The process as claimed in one or more of claims 1 to 7, wherein the mercaptan is 2-mercaptoethanol, thioglycolic acid or its methyl ester, 3-mercapto-1,2-propane-diol or 3-mercaptopropionic acid or its esters.